# EUROPEAN PATENT APPLICATION

(11) **EP 2 514 450 A1**
(43) Date of publication of application: **24.10.2012**
(21) Application number: 11163371.5
(22) Date of filing: 21.04.2011
(51) Int. Cl.: A61M 5/00, A61M 5/24, A61M 5/32

(54) **Medicated module with automatic reservoir engagement and magnetic lock mechanism**

(71) Applicant: Sanofi-Aventis Deutschland GmbH, 65929 Frankfurt (DE)
(72) Inventor: Boyd, Malcolm Stanley, Warwickshire, CV35 9PW (GB); Mercer, David Richard, Warwickshire, CV31 3BY (GB); Kouyoumjian, Garen, Warwickshire, CV31 1HN (GB)

(57) **Abstract**

A medicated module (100) for an injection system to co-deliver at least two medicaments is disclosed where a primary delivery device containing a primary medicament accepts a medicated module containing a single dose of a secondary medicament and where both medicaments are delivered through a hollow needle. The medicated module does not require the user to manually engage a reservoir containing the secondary medicament. Instead, a biasing member automatically activates the reservoir when the needle guard is retracted. The needle guard prevents accidental needle sticks before and after an injection, and locks after dose delivery. Magnet elements are provided in association with the medicated module that may be in a first, trigger locked position, and a second, triggerable position when moved by the force of another magnetic element, or by the force of the first magnet element on magnetic material.

## Description

### Field of the Present Patent Application

This invention relates medical devices and methods of delivering at least two drug agents from separate reservoirs using devices having only a single dose setting mechanism and a single dispense interface. This invention also relates to the secondary packaging in which the medical devices are stored and transported to a user. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Activation of the needle guard automatically causes the reservoir of secondary medicament to engage with dispensing conduits to allow a set dose of primary medicament and a single fixed dose of the secondary medicament to be injected. Thus, a medicated module is presented where the user does not have to manually select or set the module to dispense the second drug agent. Secondary packaging for the medicated module is designed to prevent accidental triggering of the needle guard.

### Background

Certain disease states require treatment using one or more different medicaments. Some drug compounds need to be delivered in a specific relationship with each other in order to deliver the optimum therapeutic dose. This invention is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two medicaments or active agents simultaneously. The two active agents may interact with each other during the long-term shelf life storage of the formulation. Therefore, it is advantageous to store the active components separately and only combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example, one or more actives may require a titration period to gradually introduce a patient up to a "maintenance" dose. A further example would be if one active requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more that one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir.

Providing separate storage containers for two or more active drug agents that are only combined and/or delivered to the patient during a single delivery procedure allows for the delivery of two or more medicaments in a single injection or delivery step that is simple for the user to perform. This configuration also gives the opportunity for varying the quantity of one or both medicaments. For example, one fluid quantity can be varied by changing the properties of the injection device (e.g. dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime.

This configuration also provides a medicated module that automatically causes the reservoir of secondary medicament to come into fluid communication with the primary medicament upon activation of the needle guard. This eliminates the need for the user to manually set or adjust the medicated module after performing a priming step.

To prevent the medicated module from accidental activation, the module's secondary packaging comprises a mechanism to keep the module in a locked mode. Accidental triggering may occur any time prior to use, such as during transit or storage, and may either compromise the operability of the device, or render it unusable. Factors that may cause accidental triggering may include, but are not limited to, the application of static loads (e.g., stacking, crushing), dynamic loads (e.g., impact, vibration), pack and/or device inversion or temperature fluctuation.

Where accidental triggering has the potential to compromise the integrity of the Primary Pack, a patient may be exposed to a potentially non-sterile or even harmful form of the medicament.

Our invention seeks to prevent the accidental triggering of the medicated module. The simple act of removing the medicated module from its sterile packaging takes the module from a locked stated to a triggerable state. Thus, our invention is designed in such a way that the shift in the state from "trigger locked" to "triggerable" happens automatically as part of the standard, correct use procedure.

These and other advantages will become evident from the following more detailed description of the invention.

### SUMMARY

Our invention allows complex combinations of multiple drug compounds within a single drug delivery system. The invention allows the user to set and dispense a multi-drug compound device though one single dose setting mechanism and a single dispense interface, such as a double ended needle assembly. This single dose setter controls the mechanism of the device such that a predefined combination of the individual drug compound is delivered when a single dose of one of the medicaments is set and dispensed through the single dispense interface.

By defining the therapeutic relationship between the individual drug compounds, our delivery device would help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or powders that are capable of flowing and that change shape at a steady rate when acted upon by a force tending to change its shape. Alternatively, one of the medicaments may be a solid that is carried, solubilized or otherwise dispensed with another fluid medicament.

According to one specific aspect, this invention is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment, a master or primary drug compound, such as insulin, contained within a multiple dose, user selectable device could be used with a single use, user replaceable, module that contains a single dose of a secondary medicament and the single dispense interface. When connected to the primary device, the secondary compound is activated/delivered on dispense of the primary compound. Although our invention specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyldes(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Pro-Ser-NH₂), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-lle-Glu-Trp-Leu-Lys-Asn-Gly-Gly-Pro-Ser-Ser-Gly-Ala-Pro-Pro-Ser-Lys-Lys-Lys-Lys-Lys-Lys-NH₂).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In one embodiment of our invention there is provided a medicated module attachable to a drug delivery device that comprises an outer housing having a proximal end, a distal end, and an outer surface, where the proximal end preferably has a hub holding a double-ended needle and having a connector configured for attachment to a drug delivery device. There is a reservoir in a bypass housing within the outer housing that contains a medicament. The medicated module assembly of our invention contains a needle guard that can reduce the risk of accidental needle sticks before and after use, reduce the anxiety of users suffering from needle phobia as well as preventing a user from using the device a subsequent time when the additional medicament has already been expelled.

The needle guard is preferably configured with a solid planar surface at its distal end that provides a large surface area that reduces the pressure exerted on the patient's skin, which allows the user to experience an apparent reduction in the force exerted against the skin. Preferably, the planar surface covers the entire distal end of the guard with the exception of a small needle pass through hole aligned axially with the needle. This pass through hole is preferably no more than 10 times greater in diameter than the outer diameter of the needle cannula. For example, with a needle outside diameter of 0.34mm, the pass through hole diameter D can be 4mm. Preferably, the pass through hole size should be large enough for the user to see that the device is primed (i.e., a drop or more of medicament) while not being so large that it is still possible to reach the end of the needle with a finger (i.e. needle stick injuries before or after use). This difference between the hole size and cannula diameter is to allow for tolerances, to allow users to see the drop of liquid on the end of the cannula after priming (whether a transparent or non-transparent guard is used) while keeping the size small enough to prevent accidental needle stick injuries.

Further, the movable needle guard or shield is configured to move axially in both the distal and proximal directions when pressed against and removed from an injection site. When the needle assembly is removed or withdrawn from the patient, the guard is returned to post-use extended position. A drive tooth on the inside surface of the guard engages a stop on a track on the outer surface of the bypass housing to securely lock the guard from further substantial axial movement. Preferably a lock out boss on the outer surface of the bypass housing is configured to engage a lock out feature on the inner proximal surface of the outer housing at the completion of the injection to further lock the medicated module from any further use and prevent the needle(s) and/or bypass component from being able to substantially move within the system even if the guard is held in an axially locked condition. By "substantial" movement we do not mean the typical amount of "play" in a system, but instead we mean that the guard and/or distal needle do not move axially a distance that exposes the distal end of the cannula once it is locked out.

One goal of our invention is to eliminate the need to have the user manually operate the medicated module to change the state of the module from a priming state to a combination dose delivery state. Manually operated devices are sometimes not as intuitive as they could be and raise the risk of accidental misuse. Our invention solves this problem by utilizing energy stored within the module prior to delivery of the device to the user. The stored energy can come from a biasing member, such as a compressed spring. This stored energy is released during normal user operation of the module by actuating the mechanism and thus activating the state change from prime dose to combination dose. The mechanism aims to make this actuation imperceptible to the user, consequently making the user experience of the module very similar to that of a standard commercially available and accepted needle or safety needle (i.e. unpack module, attach to a drug delivery device, prime drug delivery device, inject a set dose along with single dose in the module). In this way, the module mechanism aims to reduce the risk of unintentional misuse and to improve usability by replicating an already accepted practice for similar injection methods.

As the module mechanism does not require the user to access external features on the module for the purposes of actuation, the number of components and subsequent module size can be reduced/optimized. These factors make the mechanism ideal for a single-use, high-volume manufacture, and disposable device application. Alternatively, as the actuation is driven by a single energy source, the system lends itself to a resettable actuation mechanism. The preferred embodiment described below is the single use (non-resettable) version. The lower hub is preferably restrained rotationally with regard to the needle guard, but is free to move axially within the needle guard. The needle guard is restrained rotationally with regard to the outer housing, but is free to move axially, between defined constraints, within the outer housing.

The user pressing the distal face of the needle guard against the skin causes axial motion of the needle guard in the proximal direction. This axial motion of the guard causes a rotation of the bypass housing through the engagement and action of one or more inward-facing drive teeth on the guard as they travel in one or more drive tracks having one or more paths, which are located on the outer surface of the bypass housing. After sufficient axial travel of the needle guard, the rotation of the bypass housing brings stand-offs inside the outer housing and at the proximal ends of the lower hub into line with pockets located on the outer surface of the bypass housing. Alignment of the stand-offs with the pockets allows the bypass housing to move axially in the proximal direction and further into the outer housing. The lower hub containing a double-ended needle cannula moves axially further onto the bypass housing. Both of these movements occur due to the relaxation/release of the stored energy of the biasing member, preferably a spring that is pre-compressed during module assembly or manufacture, and constitute "triggering" of the actuation mechanism. It is this axial movement of the lower hub onto the bypass housing and the corresponding movement of the bypass housing further into the outer body that results in the double ended needles located in the distal end of the outer body and the lower hub piercing the medicated module, moving it from a state of priming to combination dose delivery.

Further axial movement of the needle guard is required in order to pierce the skin, this retraction of the needle guard temporarily further compresses the biasing member creating additional stored energy. At a "commit" point, the proximal axial movement of the drive tooth passes a non-return feature in the track through further rotation of the bypass housing. Once past this point, if the needle is removed from the skin the drive tooth is unable to return into the path it came from and must instead travel into a different path, which may be used to lock out the device. The position of the "commit" point can be adjusted through relative positioning of the paths e.g. just as the needle pierces the skin or any proportion of maximum needle insertion. If the commit point is prior to needle penetration of the user's skin, this functionality can be used to prevent cross-contamination by only allowing the needle to penetrate skin once before the device is locked out. In normal use, once the drug has been dispensed and the needle is removed from the skin, the needle guard is allowed to return axially in the distal direction under the relaxation of the biasing member as it releases its stored energy. At some point along its return travel, the drive tooth contacts a further ramped face in one of the paths of the track, resulting in yet further rotation of the bypass housing. At this point, the outer housing stand-off comes into contact with a ramp feature on the outer surface of the bypass housing. The combination of this feature with the ramp between the drive tooth and the bypass housing track results in further biasing of the bypass housing stop face into the needle guard drive tooth. The stop face features act as an axial locking pocket. The action of the combined biasing force means that any axial load in the proximal direction put on the needle guard will result in the tooth being stopped in this pocket, locking out the needle guard from further use or exposing the needle. Should the user remove the device from the skin without dispensing fluid, but after the "commit" point has been passed, the needle guard would return to an extended position and lock out as previously described.

In one embodiment of our invention there is provided a medicated module assembly attachable to a drug delivery device, preferably a pen shaped injection device, where the medicated module assembly comprises an outer housing having a proximal end and a distal end, where the proximal end has an upper hub holding a first double-ended needle cannula and a connector configured for attachment to a drug delivery device. The hub can be a separate part from the housing or integral, for example molded as part of the housing. The connector can comprise any connector design, such as threads, snap fits, a bayonet, a luer lock, or any combination thereof.

Two needle cannulae may be used, a distal cannula and a proximal cannula, with both cannulae preferably being doubled-ended for piercing a septum or seal and for piercing skin. The distal needle is mounted in a lower hub and the proximal needle is mounted in the upper hub, each using any technique known to those skilled in the art, such as welding, gluing, friction fit, over-molding and the like. The medicated module assembly also contains a biasing member, preferably a torsion/tension/compression spring. The biasing member is preferably assembled in an energized state and positioned between the proximal inner face of the needle guard and the distal face of the lower hub. For a compression spring this would mean that the spring is assembled into the device pre-compressed state, within an axial space shorter than its free length. Although a preferred biasing member is a spring, any type of member that produces a biasing force will work.

The medicated module assembly of our invention automatically, once triggered, changes state from (1) a pre-use or priming state, where a small amount of primary medicament flows in a bypass around the reservoir containing a single dose of the secondary medicament, to (2) a ready-to-use or combination dose state, where both the upper and lower cannulae are in fluidic engagement with the fixed dose of the second medicament within the module and where a set dose of the primary medicament can be injected along with the non-settable single dose of secondary medicament in the reservoir, and finally to (3) a locked out state, where the needle guard is prevented from substantial proximal movement. The outer housing preferably has a window or indicator that shows the various states of the module. The indicator can be a pip, knob, button, or the like that protrudes through the outer surface of the proximal end of the needle guard and visually shows the user whether the module is in the pre-use or ready-to-use state. It may also be a visual indicator, e.g. showing colors or symbols, or a tactile or audible indicator. Preferably, user noticeable indicia may indicate both a pre-use priming position and a locked position of the guard after the medicated module assembly has been used to perform an injection.

Inside the bypass housing there is a cavity that contains the capsule, which comprises the single dose of medicament in the reservoir. As the needle guard is retracted during an injection, the bypass housing is moved proximally along with the capsule positioned inside the cavity. This allows the seals of the capsule to be pierced at its top and bottom by the needle cannula such that the medicament can be expelled from the reservoir during dose delivery. When connected to a drug delivery device containing a first medicament and prior to piercing the seals of the reservoir, the needle cannulae are only in fluid communication with the first medicament and a fluid flow path that bypasses the capsule. Preferably, a channel on the inside surface of the bypass housing is part of this fluid flow path and is used in the priming function of the drug delivery device. This allows the primary device to be primed through the medicated module by the user without dispensing the contents of the medicament reservoir inside the medicated module.

As mentioned, the bypass housing preferably has one or more tracks located on the outside surface each having a set of first, second, third, and fourth paths. On the inner surface of the proximal end of the needle guard is one or more radial protrusions or drive teeth. As the guard first begins to retract, these protrusions travel in the first path, causing the bypass housing to slightly rotate. As the guard continues to retract and then partially extend, the protrusions travel in the second and third paths. The protrusion moves to the fourth path and into a locking position when the guard is fully extended to its post-use position, which is preferably less extended than the starting position to provide an indication that the device has been used. The guard is rotationally constrained by the outer housing, preferably by the use of one or more spline features in the outer surface of the guard in cooperation with one or more followers or pips located at the distal end of the inner surface of the outer housing. The bypass housing is rotationally constrained when the protrusion is in the second path of the track. As the protrusion is moved axially in the proximal direction when the guard retracts, the protrusion moves from the second track to the third track.

A further aspect of the invention relates to a method of dispensing a fixed dose of one medicament and a variable dose of a primary medicament from separate reservoirs that involves the steps of first attaching a medicated module to a delivery device set in a pre-use or prime only state. The user can prime the dose delivery device using only the primary medicament and bypassing the second medicament. After priming the user begins the injection and the needle guard begins to retract and the module automatically changes to second state that allows a combination delivery of the two medicaments. Upon completion of the delivery procedure and retraction of the needle from the injection site, the extension of the needle guard automatically changes the module to a third state.

In one arrangement, during dispense, substantially the entire amount of second medicament can be expelled as well as the selected or dialed dose of the first medicament, through the single dispense interface. The capsule preferably contains a flow distributor to ensure that substantially all the single dose of secondary medicament is forced out of the capsule by the primary medicament during an injection. The flow distributor can be a separate stand alone insert or pin, or it may be integral with the capsule to make a one piece component utilizing, for example, design principles such as form fit, force fit or material fit, such as welding, gluing, or the like, or any combination thereof. The one-piece component may comprise one or more medicament flow channels, preferably one flow channel. The flow distributor can be constructed of any material that is compatible to the primary and secondary medicaments. A preferred material is one that is typically used to manufacture septa or pistons (bungs) found in multi-dose medicament cartridges, however, any other material that is compatible with the drug could be used, e.g., glass, plastics or specific polymers as described below. By "substantially all" we mean that at least about 80% of the second medicament is expelled from the drug delivery device, preferably at least about 90% is expelled. In the third state, preferably the module is locked so as to prevent a second delivery or insertion by means of a locking mechanism as described previously.

The combination of compounds as discrete units or as a mixed unit is delivered to the body via an integral needle. This would provide a combination drug injection system that, from a user's perspective, would be achieved in a manner that very closely matches the currently available injection devices that use standard needles.

The medicated module of our invention can be designed for use with a drug delivery device with an appropriate compatible interface. However, it may be preferable to design the module in such a way as to limit its use to one exclusive primary drug delivery device (or family of devices) through employment of dedicated/coded/exclusive features to prevent attachment of a non-appropriate medicated module to a non-matching device. In some situations it may be beneficial to ensure that the medicated module is exclusive to one drug delivery device while also permitting the attachment of a standard drug dispense interface to the device. This would allow the user to deliver a combined therapy when the module is attached, but would also allow delivery of the primary compound independently through a standard drug dispense interface in situations, such as, but not limited to, dose splitting or top-up of the primary compound.

A particular benefit of our invention is that the medicated module makes it possible to tailor dose regimes when required, especially where a titration period is necessary for a particular drug. The medicated module could be supplied in a number of titration levels with obvious differentiation features such as, but not limited to, aesthetic design of features or graphics, numbering etc, so that a patient could be instructed to use the supplied medicated module in a specific order to facilitate titration. Alternatively, the prescribing physician may provide the patient with a number of "level one" titration medicated modules and then when these were finished, the physician could then prescribe the next level. A key advantage of this titration program is that the primary device remains constant throughout.

In a preferred embodiment of our invention, the primary drug delivery device is used more than once and therefore is multi-use; however, the drug delivery device may also be a single use disposable device. Such a device may or may not have a replaceable reservoir of the primary drug compound, but our invention is equally applicable to both scenarios. It is also possible to have a suite of different medicated modules for various conditions that could be prescribed as one-off extra medication to patients already using a standard drug delivery device. Should the patient attempt to reuse a previously used medicated module, our invention includes the locking needle guard that is activated after a first predefined travel/retraction of the guard/insertion of the needle. The locked needle guard would alert the patient to this situation and the inability to use the module for a second time. Visual warnings (e.g. change in color and/or warning text/indicia within an indication window on the module once insertion and/or fluid flow has occurred) and/or a change in the shape or length of the device as described previously can also be used. Additionally, tactile feedback (presence or absence of tactile features on the outer surface of the module hub following use) could be used as well.

A further feature of our invention is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

Our invention also covers a method of delivering two fluids (such as two medicaments or a buffer and/or a solvent) stored in separate primary packages. The medicaments may both be liquid, or alternatively one or more of the medicaments may be a powder, suspension or slurry. In one embodiment the medicated module could be filled with a powdered medicament that is either dissolved or entrained in the primary medicament as it is injected through the medicated module.

Furthermore, our invention is also directed to secondary packages for storing and transporting the medicated modules. The secondary packages are designed with magnetic elements (e.g., magnets or magnetic material) that pull pins on the medicated module into an "interference state," or a "trigger locked state," wherein the pins are positioned so as to be in interference with the needle guard or body of the module, thus preventing the relative motion between the needle guard and the device body. When the module is removed from the secondary packaging and the proximity to the magnetic field is lost, the pins relax under release of springs attached to the pins, moving out of interference between the needle guard and device body and thus transitioning the module to a "triggerable" state where it is ready to be used.

In another embodiment, a medicated module comprises a spring, which is attached to a wall within the module at one end, and is attached to a magnetic pin at the other end. In the rest state, the magnetic pin extends into an indent in a rotary element within the module, preventing the internal components from rotating. When the internal components are prevented from rotating, the module is in a trigger locked state, as the rotation of the components is required to trigger the module. Insertion of the distal end of a drug delivery device, comprising a magnetic element at or near the distal end, pulls the magnetic pin upward toward the magnetic element, and out of the indent in the rotary element. Once the magnetic pin is fully removed from the indent, the rotary element is free to rotate, and the module is thus in the triggerable position.

In yet another embodiment, a medicated module within a secondary packaging, and a drug delivery device to attach to the medicated module are provided. On the interior surface of the secondary packaging a first end of a spring is attached. The second end of the spring is attached to a magnetic pin. The magnetic pin is affixed to an indent within the medicated module, and retains the medicated module in place within the secondary packaging. Once the drug delivery device, which contains a magnetic element at its distal end, is inserted into the medicated module, the magnetic field emitted by the magnetic element repels the magnetic pins, moving them out from within the indents and thus freeing the module from the secondary packaging. One purpose of such an arrangement is to ensure that a stored energy present in the medicated module, such as an energized biasing member described above, is protected from external interference and minimizes the likelihood of accidental triggering of the device and release of this stored energy up until the point that the user removes it from its packaging for use.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates one possible drug delivery device that can be used with the present invention;
Figure 2 illustrates an embodiment of the medicated module of the present invention, where the medicated module is separated from an attachable cartridge holder of drug delivery device;
Figure 3 illustrates an exploded distal perspective view of all the components (except the medicated capsule) of the medicated module illustrated in Figure 2;
Figure 4 illustrates an exploded proximal perspective view of all the components (except the medicated capsule) of the medicated module illustrated in Figure 2;
Figure 5 is a perspective view of the capsule containing the reservoir of the embodiment of Figure 2;
Figure 6 illustrates a proximal perspective view of the outer housing of the embodiment of Figure 2;
Figure 7 is a sectioned view of the embodiment of the medicated module shown in Figure 2 orientated in the bypass configuration;
Figure 8 is a close-up perspective view of the bypass housing of the embodiment of the medicated module shown in Figure 2 to illustrate the positions of the drive tooth during use;
Figure 9 is a cross-sectional view of an exemplary medicated module within a secondary packaging in a trigger locked or interference position;
Figure 10 is a close-up view of the medicated module within the secondary packaging of Figure 9;
Figure 11 is a cross-sectional view of the medicated module and the secondary packaging of Figure 9, wherein the module is being removed from the packaging and is in a triggerable position;
Figure 12 is a cross-sectional view of an exemplary medicated module in a trigger locked or interference position;
Figure 13 is a cross-sectional view of the medicated module of Figure 12 and a primary device that is inserted into the medicated module; and
Figure 14 is a cross-sectional view of an exemplary medicated module within a secondary packaging in a trigger locked or interference position, and a primary device with a magnetic element to be inserted into the medicated module.

### DETAILED DESCRIPTION

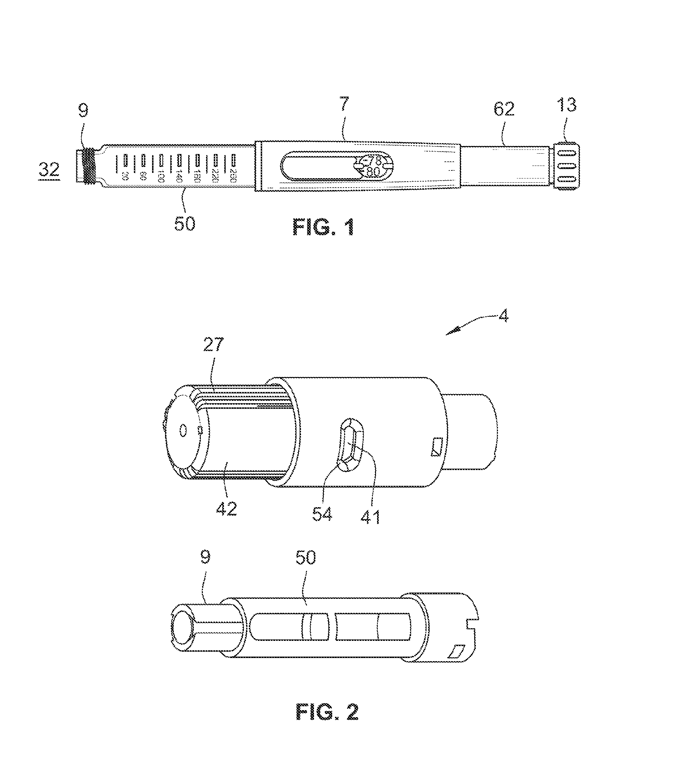

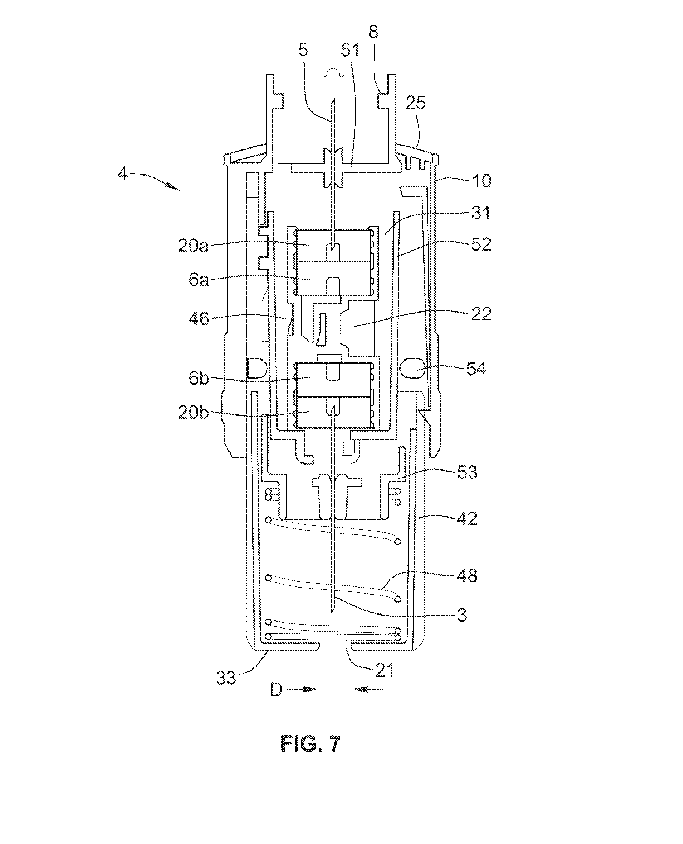

The present invention provides a locking mechanism for a medicated module and secondary packaging for the medicated module. The medicated module administers a fixed predetermined dose of a secondary drug compound (medicament) along with a variable dose of a primary or first drug compound through a single output or drug dispense interface. Setting the dose of the primary medicament by the user determines the settable dose of the primary medicament. A fixed dose of the second medicament, which preferably is a single dose contained in a capsule or reservoir having an integral flow distributor, may be administered along with the settable dose of the primary medicament. In a preferred embodiment the drug dispense interface is a needle cannula (hollow needle). Fig. 1 illustrates one example of a drug delivery device 7 that the medicated module 4 (see Figs. 2 or 7) can be attached to. The medicated module can be attached by the connection means 9 on distal end 32 of cartridge holder 50. Each medicated module is preferably self-contained and provided as a sealed and sterile disposable module that has an attachment means 8 compatible to the attachment means 9 at the distal end 32 of device 7.

Any known attachment means 8 can be used to attach the medicated module to the chosen drug delivery device, including all types of permanent and removable connection means, such as threads, snap locks, snap fits, luer locks, bayonet, snap rings, keyed slots, and combinations of such connections. Figs. 2, 4, and 7 illustrate the attachment means 9 as a unique bayonet type connection that is keyed specifically to a corresponding female bayonet type connection 8 on hub 51 of medicated module 4. The embodiments shown in Figs. 2, 4, 5, and 7 have the benefit of the second medicament as a single dose being contained entirely within capsule 31, and specifically in reservoir 22, hence minimizing the risk of material incompatibility between the second medicament and the materials used in the construction of the medicated module 4, specifically housing 10, inner housing 52, or any of the other parts used in the construction of the medicated module.

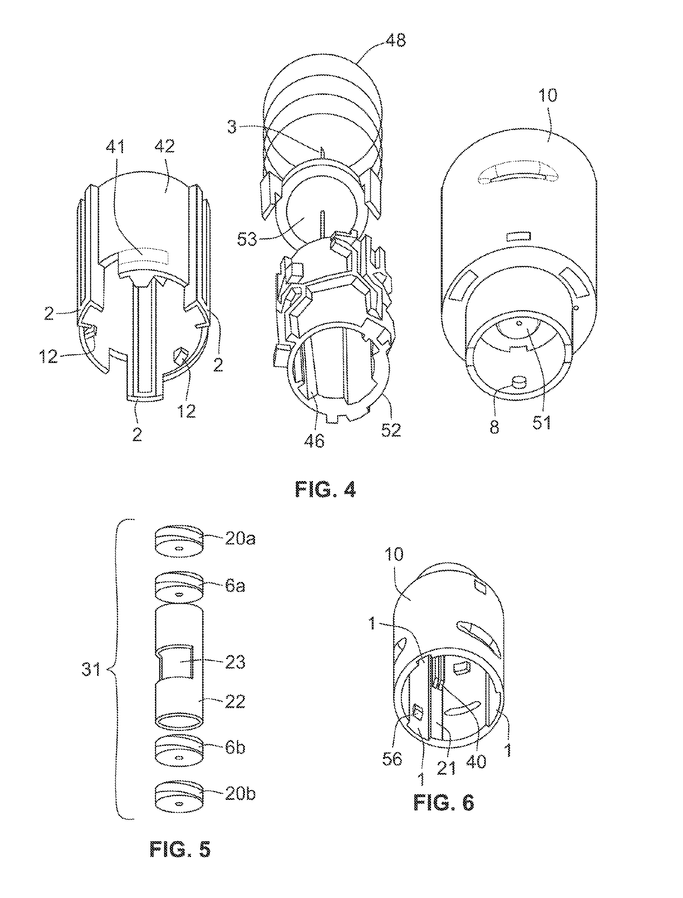

To minimize the residual volume of the second medicament, caused by recirculation and/or stagnant zones, that might remain in capsule 31 at the end of the dispense operation, it is preferable to have a flow distributor 23 as an integral part of reservoir 22 (see Fig. 5). The reservoir 22 containing the single dose of the secondary medicament can be sealed with septa 6a and 6b, which are fixed to the capsule using keepers or plugs 20a and 20b. Preferably the keepers have fluid channels that are in fluid communication with needles 3 and 5 and with bypass 46, which is preferably part of the inside surface of bypass housing 52. Together this fluid path allows priming of the drug delivery device before injection. Preferably the reservoir, flow distributor, keepers, and bypass can be made from materials that are compatible with the primary medicament. Examples of compatible materials of construction include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). The needle pierceable septa, bungs, and/or seals that are used with both the capsule and the primary medicament cartridge can be manufactured using TPE (thermo plastic elastomer); LSR (liquid silicone rubber); LDPE (low density polyethylene); and/or any kind of medical grade rubber, natural or synthetic.

The design of flow distributor 23 should ensure that at least about 80% of the second medicament is expelled from reservoir 22 through the distal end of needle 3. Most preferably at least about 90% should be expelled. Ideally, displacement of the first medicament in a primary reservoir (not shown) contained in cartridge holder 50 and through the capsule 31 will displace the single dose of the second medicament stored in reservoir 22 without substantial mixing of the two medicaments.

Attachment of the medicated module 4 to the multi-use device 7 causes proximal needle 5 to penetrate a septum (not shown) sealing the distal end of the cartridge of primary medicament positioned in cartridge holder 50 of the multi-use device 7. Once needle 5 has passed through the septum of the cartridge, fluid connection is made between the first medicament and the needle 5. At this point, the system can be primed by dialing out a small number of units (or cocking the device if only a single dose selection is possible) using dose dial sleeve 62. Once the device 7 is primed, then activation of the needle guard 42 allows dispense of the medicaments by subcutaneously injecting the medicaments via activation of a dose button 13 on device 7. The dose button of our invention may be any triggering mechanism that causes the dose of the first medicament that was set by the dose dial sleeve 62 to move towards the distal end 32 of the device. In a preferred embodiment the dose button is operably connected to a spindle that engages a piston in the primary reservoir of the first medicament. In a further embodiment the spindle is a rotatable piston rod comprising two distinct threads.

One embodiment of the medicated module 4 is illustrated in Figs. 2 and 7. In these embodiments the medicated module 4 contains a capsule 31 comprising a reservoir 22, two keepers 20a and 20b, and two seals 6a and 6b. Reservoir 22 contains a fixed single dose of a secondary medicament. In some cases this secondary medicament may be a mixture of two or more drug agents that can be the same or different from the primary drug compound in the drug delivery device 7. Preferably the capsule is permanently fixed within the medicated module, however, in some cases it may be preferred to design the module such that the capsule can be removed when empty and replaced with a new capsule.

In the embodiments shown in Figs. 5 and 7, capsule 31 has ends that are sealed with pierceable membranes or septa 6a and 6b that provide a hermetically sealed and sterile reservoir 22 for the second medicament. A primary or proximal engagement needle 5 can be fixed in hub 51 connected to the proximal end of housing 10 of the module and configured to engage capsule 31 when the bypass cavity is moved in the proximal direction by the action of the needle guard and biasing member during triggering. The outlet, or distal needle 3, is preferably mounted in lower hub 53 and initially protrudes into lower keeper 20b. The proximal end of needle 3 pierces the lower septum 6b when the bypass housing 52 rotates and is moved proximally by the force exerted by needle guard 42 and spring 48 during injection.

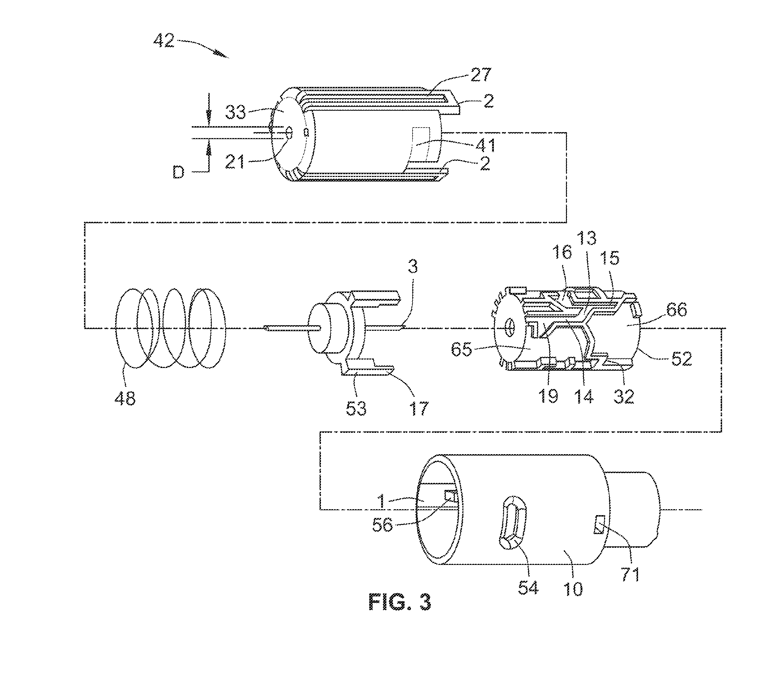

When first attached to the delivery device, the medicated module 4 is set at a pre-use or starting position. Preferably, indicator 41 shows through window 54 to inform the user of the pre-use condition of the medicated module. The indicator is preferably a color stripe or band on the outer surface of the proximal end of guard 42 (see Fig. 3) visible through an aperture in the outer body. The needle guard 42 is slidably engaged with inner surface of outer housing 10 by engagement of splines 2 and channels 1. Retention snaps 56 prevent the guard from disengaging the outer housing at its fully extended position. Housing 10 partially defines an internal cavity 21 that holds bypass housing 52, which contains capsule 31. A portion of the proximal end of housing 10 defines an upper hub 51 that holds needle 5. Optionally, as illustrated in Fig. 7, a shoulder cap 25 may be added to the proximal outer surface of outer housing 10. This shoulder cap can be configured to serve as indicia to identify to a user the type/strength of medicament contained in the module. The indicia can be tactile, textual, color, taste or smell.

Fig. 7 shows a cutaway or cross-sectioned view of the medicated module set in a pre-use or starting state where needles 3 and 5 are not piercing septa 6a and 6b. In this position, the bypass housing 52 is at its most extended position and needles 3 and 5 are not in fluid communication with medicament contained in capsule 31. The capsule is supported by bypass housing 52. In this neutral or suspended state of capsule 31, primary medicament from the cartridge in cartridge holder 50 of device 7 can flow through needle 5 into keeper 20a, through bypass 46 and into keeper 20b, and eventually out through needle 3. This flow configuration allows a user to perform a priming step or procedure by setting a small dose of the primary medicament using the dose dial sleeve 62 and dose button 13 on the drug delivery device 7.

The compression spring 48 is positioned between the distal end of bypass housing 52 and the inner distal face of guard 42 to bias the guard 42 into an extended (guarded) position as illustrated in Fig. 7. Upon assembly, spring 48 is purposely compressed to supply a proximally directed biasing force against lower hub 53. This pre-compression of spring 48 is possible because the lower hub 53 and the bypass housing 52 are prevented from moving in an axial proximal direction by radial stand off 40 located on the inside surface of the outer housing (Fig.6) that engage with an upper stand off pocket 66 and legs 17 of lower hub 53 engaging lower stand off pocket 65. The combination of these stand-offs/legs and pockets prevent the lower hub and upper hub needles from piercing into the centre of the capsule until the device is triggered as previously described.

The proximal inside surface of guard 42 has one or more inwardly protruding features, drive teeth, pips, or like structures 12 that run in one or more tracks 13 or guide ways formed in the outer surface of bypass housing 52. As shown in Fig. 3, track 13 can be described as four paths, 19, 14, 15, and 16, that have a specific geometry such that after a single use of the medicated module 4 the drive tooth 12 is blocked from further axial movement and the guard (and device) is "locked" in a guarded position where the distal end of the needle is completely and safely covered by guard 42.

One potential feature of our medicated module assembly is the user feedback that may be given when the assembly is used. In particular, the assembly could emit an audible and/or tactile "click" to indicate to the user that they have triggered the device. This audible and/or tactile feature could work as follows. As mentioned, the needle guard 42 is rotationally constrained by outer housing 10 and has one or more drive teeth 12 that are initially in path 19 of track 13 on bypass housing 52. As the guard is moved proximally, the spring 48 is further compressed exerting additional force in the proximal direction on lower hub 53, which is initially constrained axially by the lower stand off pocket 65 engaged with legs 17. Likewise, the bypass housing 52 is constrained from moving proximally by upper stand off pocket stop 132 engaged with stand off 40 on the inner surface of outer housing 10. The drive teeth 12 travel in path 19 causing the bypass housing to rotate slightly. This rotation will disengage the upper stand off 40 from upper standoff pocket stop 132, allows the drive teeth to align with path 14, and unblocks legs 17 from lower standoff pocket allowing the bypass housing to move proximally carrying with it capsule 31, where it then can engage needles 3 and 5. This movement can occur rapidly under the force provided by the relaxation of the spring 48 and as such can emit an audible "click" sound as the proximal face of the bypass housing 52 contacts the inner proximal face of housing 10 and the proximal face of lower hub 53 contacts distal face of housing 52.

As the guard continues to move proximally, the drive teeth move from path 14 passed transition point 14a into path 15 causing further rotation of the bypass housing. This transition past point 15a (and the corresponding point directly below it on the track) constitute the "commit" point and as such, once it has been reached the needle guard 42 will "lock out" when it extends upon removal of the device from the injection site.

As mentioned, the distal end of the guard 42 has a planar surface 33 that provides an added measure of safety and reduces the pressure exerted by the guard on the injection site during an injection with our needle assembly. Because the planar surface 33 substantially covers access to needle 3 a user is prevented from gaining access to the distal tip of the needle after the assembly is in the locked position. Preferably, the diameter D of needle pass through hole 21 in the planar surface is no more than 10 times that of the outer diameter of needle cannula 3.

The outer proximal surface of the needle guard 42 preferably has indicia 41 that are preferably at least two different color stripes or bands, each of which is sequentially visible through the opening or window 54 in outer housing 10. One color could designate the pre-use or prime state of the module and the other color would indicate that the module is in finished or locked state, another color could be used to denote the transition through the trigger or "commit" point in case a user stops injection after trigger point but before "commit" point. For example, a green color could be the pre-use position and a band of red color could be used to indicate that the module has been used and is locked and an orange color could indicate that the device has been triggered but not locked out. Alternatively, graphics, symbols or text could be used in place of color to provide this visual information/feedback. Alternatively these colors could be displayed using the rotation of the bypass cavity and printed on or embedded into the bypass housing. They could be visible through the aperture by ensuring that he needle guard is made form a transparent material.

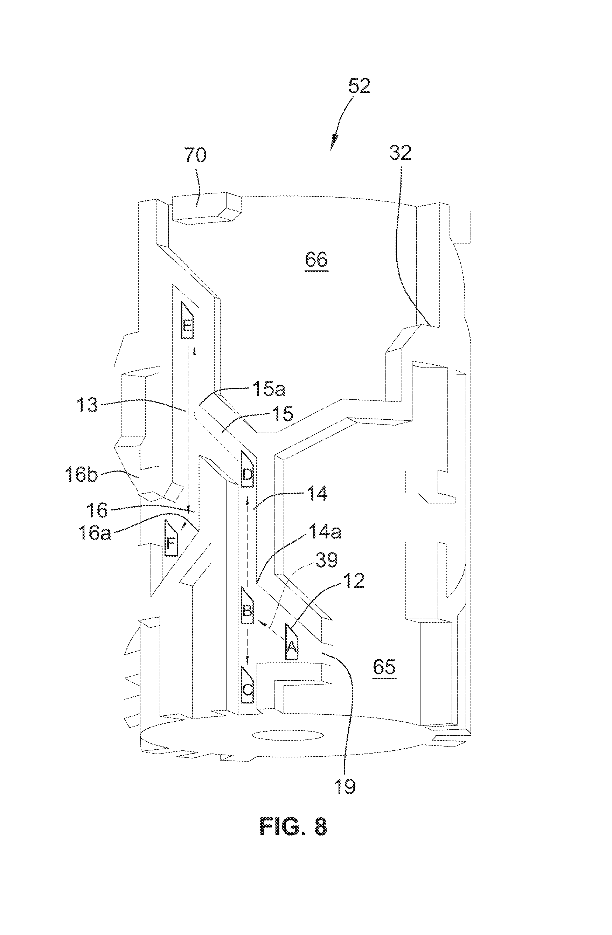

Fig. 8 illustrates the travel of drive teeth 12 in one or more tracks 13 as illustrated by directional arrow 39. Drive tooth 12 begins at position A and through axial movement of the needle guard, biases the bypass housing rotationally until it moves past the transition point 14a and arrives at position B. Once the drive tooth reaches position B, the bypass housing and lower needle hub move proximally causing the capsule 31 to engage needles 3 and 5, and the drive tooth moves relatively to position C (this is termed as the triggering of the device) and it is the bypass housing/lower hub moving proximally under the release of stored energy that results in the effective position of the needle guard drive tooth being position C. It is important to note that the needle guard does not move under the action of the release stored energy, it is just the needle hub and the bypass housing that move relatively away from the needle guard at the point of triggering, hence the drive tooth moves from position B to position C. As the needle guard continues to retract, drive tooth 12 moves proximally in path 14 to position D, where it exerts a rotational bias on the bypass housing 52, causing it to rotate again until tooth 12 passes the transition 15a (commit point) into path 16. The axial position of the commit point 15a with regards to the resultant position of the needle guard 42 relative to the tip of the distal needle 3 can be adjusted so as to determine the length of needle allowed to be inserted into the user's skin prior to locking out of the device. This can be used to prevent cross contamination and re-use of the needle or to allow users to insert the cannula 3 a small depth into the skin without committing the device so as to allow the user to find an acceptable injection site prior to full insertion.

The drive tooth then moves proximally until position E is reached. At this point, the needle guard 42 is fully retracted and the full available insertable length of the needle is exposed. Once the user removes the guard from contact with the skin, the guard begins to extend as a result of the distal biasing force exerted by spring 48 on the inner proximal surface of the guard. The utilization of the stored energy spring to act both as a trigger/piercing spring and also, once extended post triggering, as the needle guard spring, is a unique aspect of this design. It negates the need to use two separate springs for these separate functions by locating the spring in a position such that it can fulfill both roles. Initially, for example during assembly or manufacture of the medicated module, the biasing member is compressed, exerting a force on the lower hub/bypass housing in preparation for triggering. Once triggered it extends proximally where upon it can then be compressed from the distal end as the needle guard retracts against it. This secondary compression provides the force to push the needle guard back to the extended and locked position as it is removed from the injection site. As the guard moves to its fully extended post-use position, which preferably is less extended than the starting position, the drive tooth 12 moves distally in path 15 until it reaches transition point 16a, where it then rotationally biases the bypass housing 52 to rotate yet again until tooth 12 enters path 16 and arrives at position F. This last rotation of bypass housing 52 causes lock out boss 70 to engage lock out feature 71. This prevents any further axial movement of the bypass housing. The needle guard is prevented from further substantial axial movement, as defined earlier, by engagement of the drive tooth with axial stop 16b. It is within the scope of our invention that a number of tooth arrangements and/or profiles could be used to fulfill the required function described above, e.g., simple equal tooth profiles or more complex multi-angled profiles. The particular profile being dependent upon the required point of commit and rotation of the bypass housing. It is also within the scope of our invention that a similar axial/rotational locking of the lower needle hub to the bypass housing as of the bypass housing to the outer housing, could be integrated to prevent movement of the needle post-triggering and post-lock out.

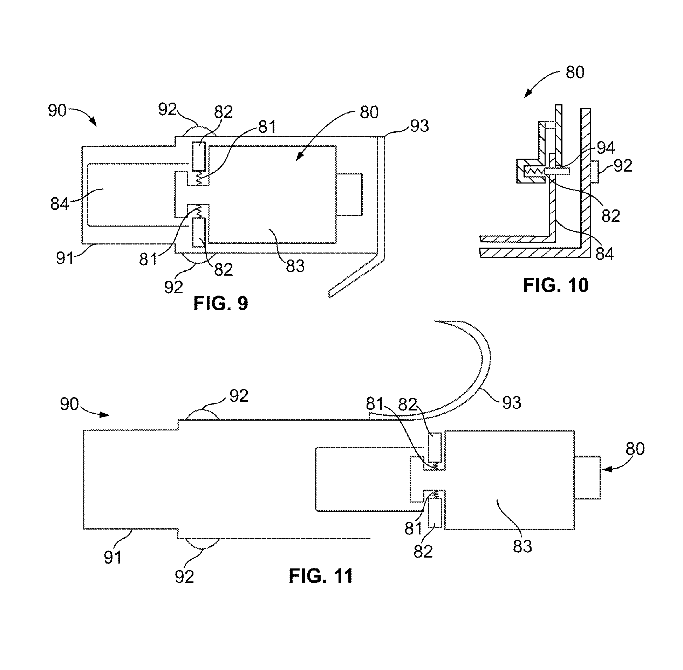

Fig. 9 is a cross-sectional view of an exemplary medicated module 80 within a secondary packaging 90, in a trigger locked or interference position, such as the medicated module 4 illustrated in Figs. 2 and 7.

In this embodiment, medicated module 80 may comprise at least some of the same components as those described for the medicated module 4 of Figs. 2 and 7. In addition, the medicated module 80 comprises a plurality of springs 81 and magnetic pins 82. There are preferably two springs 81 and two magnetic pins 82. A first end of each spring 81 is attached to a housing 83 of the medicated module 80, and a second end is attached to magnetic pin 82.

Secondary packaging 90 comprises a main body 91, a plurality of magnet elements 92 present on the main body 91, and a lid 93. There are preferably two elements 92 on main body 91. The magnet elements 92 may be located on the exterior surface or the interior surface of main body 91, or may extend from the exterior surface to the interior surface.

When the medicated module 80 is placed within or in proximity to secondary packaging 90, magnets 92 attract magnetic pins 82, such that magnetic pins 82 are pulled in the direction toward magnets 92. Magnetic pins 82 may extend as close to magnets 92 as springs 81 allow. When the magnetic pins 82 are extended toward magnets 92, the magnetic pins 82 block the edges of the needle guard 84. Axial travel of the needle guard 84 is thus prevented due to interference of the needle guard 84 edges with magnetic pins 82. This allows the interfering magnetic pins 82 to maintain the medicated module 80 in a locked, non-triggerable position.

Fig. 10 is a close-up view of the medicated module within the secondary packaging of Fig. 9. In this view, the pull of the magnetic pin 82 toward the magnet 92 is shown, and the magnetic field emitted by magnet 92 is represented in part by lines 94. As can be seen in Fig. 10, needle guard 84 is blocked from axial movement by magnetic pin 82. One advantage of such a configuration is that is prevents axial movement of the drive tooth which would cause rotation of bypass cavity and hence triggering of the device.

Fig. 11 is a cross-sectional view of the medicated module 80 and the secondary packaging 90 of Fig. 9, wherein the module 80 is being removed from the packaging and is in a triggerable position. As the module 80 is removed from secondary packaging 90, magnetic pins 82 are no longer near the magnetic field emitted by magnets 92, and as a consequence are not pulled toward magnets 92. Once the proximity to the magnetic field 94 is lost, magnetic pins 82 relax under the release of the tension in springs 81, moving to the position shown in Fig. 11. In this position, the magnetic pins 92 no longer extend so as to block needle guard 84. This is the "triggerable" position, in which the medicated module may be activated for use because the needle guard 84 is free to move axially toward housing 83. As such, one advantage of such an arrangement is that it allows the drive tooth to hit the drive path on the bypass cavity.

The system illustrated in Figs. 9-11 may be re-usable such that whenever medicated module 80 is inserted into secondary packaging 90, the magnetic field 94 pulls magnetic pins 82 toward magnet 92.

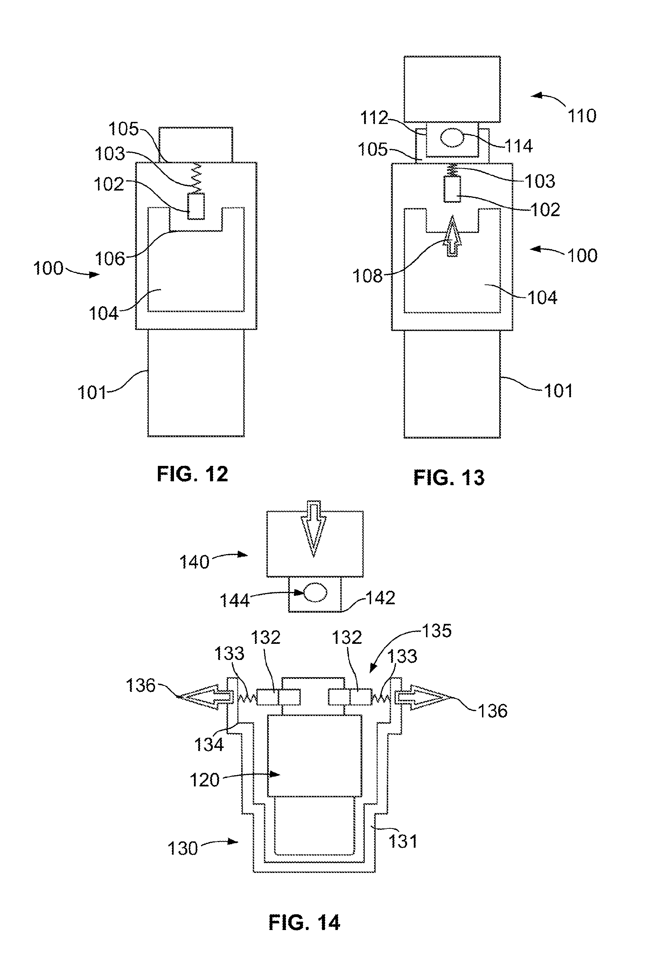

Fig. 12 is a cross-sectional view of an exemplary medicated module 100 in a trigger locked or interference position. Medicated module 100 comprises a main body 101, a magnetic pin 102, a spring 103, and a rotary element 104. A first end of spring 103 is attached to a wall 105 within main body 101. A second end of spring 103 is attached to magnetic pin 102. Rotary element 104 comprises an indent 106.

In the trigger locked position, magnetic pin 102 extends into indent 106 in rotary element 104. Spring 103 pushes pin 102 down into indent 106, in the position shown in Fig. 12.

Fig. 13 is a cross-sectional view of the medicated module 100 of Fig. 12, the module being attached to a drug delivery device 110. In Fig. 13, only the distal end 112 of drug delivery device 110 is shown. Drug delivery device 110 may comprise the same or similar features as the drug delivery device 7 illustrated in Fig. 1. For example, an attachment means may be present on the cartridge holder of drug delivery device to affix the device 110 to medicated module 100. Device 110 also comprises a magnet 114 near its distal end 112.

Once the distal end 112 of device 110 is inserted into the hub of medicated module 100, magnet 114 is in proximity to magnetic pin 102. The magnetic field exerted by magnet 114 draws magnetic pin 102 in the direction shown by arrow 108, and out of indent 106. When magnetic pin 102 is completely removed from the walls that define indent 106, bypass cavity 104 is free to move, and the module 100 is in a "triggerable" state.

Fig. 14 is a cross-sectional view of an exemplary medicated module 120 within a secondary packaging 130, and a drug delivery device 140.

Drug delivery device 140 may comprise the same or similar features as the drug delivery device 7 illustrated in Fig. 1. For example, an attachment means may be present on the cartridge holder of drug delivery device to affix the device 140 to medicated module 100. Device 140 also comprises a magnet 144 near its distal end 142.

Secondary packaging 130 comprises a main body 131, a plurality of magnetic pins 132, and a plurality of springs 133. Secondary packaging 130 also comprises an open end 135, through which a medicated module may be inserted and removed. A first end of each spring 133 is attached to a wall 134 within main body 131. A second end of each spring 133 is attached to each of the magnetic pins 132. In the trigger locked position, at least a portion of each magnetic pin 132 resides within an indent in main body 120. Fig. 14 shows the magnetic pins 132 within indents along the sides of main body 120. One function of such magnetic pins is that they block the relative axial movement and as such, prevent drive tooth action on the bypass cavity.

When drug delivery device 140 is inserted into medicated module 120, the magnetic field emitted by magnet 144 will repel the magnetic pins 132, and the magnetic pins 132 will move out of the indents and away from the medicated module, in the direction of the arrows 136. Once the magnetic pins 132 are removed from the indents in the medicated module 100, the module can be removed from the secondary packaging 130.

In any of the above described embodiments of our invention, the second medicament may be either in a powdered solid state, any fluid state contained within the secondary reservoir or capsule. The greater concentration of the solid form of the medicament has the benefit of occupying a smaller volume than the liquid having lower concentration. This in turn reduces the ullage of the medicated module. An additional benefit is that the solid form of the second medicament is potentially more straightforward to seal in the secondary reservoir than a liquid form of the medicament. The device would be used in the same manner as the preferred embodiment with the second medicament being dissolved by the first medicament during dispense.

Preferably the medicated module is provided by a drug manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user. Features such as angled surfaces on the end of the injection device or features inside the module may assist this opening of the seal.

The medicated module of our invention should be designed to operate in conjunction with an injection device, preferably a pen-type multi-dose injection device, similar to what is illustrated in Fig. 1. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose and preferably a multi-dose device, however, in some cases it may be beneficial to use a single dose, disposable device.

A typical injection device contains a cartridge or other reservoir of primary medication. This cartridge is typically cylindrical in shape and is usually manufactured in glass. The cartridge is sealed at one end with a rubber bung and at the other end by a rubber septum. The injection device is designed to deliver multiple injections. The delivery mechanism is typically powered by a manual action of the user, however, the injection mechanism may also be powered by other means such as a spring, compressed gas or electrical energy. In a preferred embodiment, the delivery mechanism comprises a spindle that engages a piston in the reservoir. In a further embodiment the spindle is a rotatable piston rod comprising two distinct threads.

Exemplary embodiments of the present invention have been described. Those skilled in the art will understand, however, that changes and modifications may be made to these embodiments without departing from the true scope and spirit of the present invention, which is defined by the claims.

List of references
- 1: channels
- 2: engagement arms
- 3: distal needle
- 4: medicated module
- 5: proximal needle
- 6a: top septum / membrane / seal
- 6b: bottom septum/ membrane / seal
- 7: drug delivery device
- 8: attachment means / connector
- 9: connection means/ attachment means
- 10: housing
- 12: drive tooth
- 13: track
- 14: path
- 14a: transition point
- 15: path
- 15a: transition point
- 16: path
- 16a: transition point
- 16b: axial stop
- 17: legs
- 19: path
- 20a, 20b: keepers
- 21: hole

- 22: reservoir
- 23: flow distributor
- 25: shoulder cap
- 31: capsule
- 32: distal end of device
- 33: planar surface
- 39: path/directional arrow
- 40: radial stand off
- 42: guard
- 46: bypass
- 48: spring/biasing member
- 50: cartridge holder
- 51: upper hub
- 52: bypass housing
- 53: lower hub
- 54: window
- 56: retention snap
- 62: dose setter/dose dial sleeve
- 65: lower stand off pocket
- 66: upper stand off pocket
- 70: lock out boss
- 71: lock out feature
- 132: upper stand off pocket stop
- 80: medicated module
- 81: spring
- 82: magnetic pin

- 83: housing
- 84: needle guard
- 90: secondary packaging
- 91: main body
- 92: magnet
- 93: lid
- 94: lines
- 100: medicated module
- 101: main body
- 102: magnetic pin
- 103: spring
- 104: rotary element
- 105: wall
- 106: indent
- 108: arrow
- 110: drug delivery device
- 112: distal end
- 114: magnet
- 120: medicated module
- 130: secondary packaging
- 131: main body
- 132: magnetic pin
- 133: spring
- 134: wall
- 135: open end
- 136: arrows

- 140: drug delivery device
- 142: distal end
- 144: magnet

## Claims

1. A medicated module (4, 100) attachable to a drug delivery device (110), comprising,
a. an outer housing (10) having an inner surface, a proximal end and a distal end, where the proximal end has an upper hub holding a first dispense interface (5) and a connector (9) configured for attachment to a drug delivery device (110);
b. a bypass housing (52) having an outer surface and slidably engaged with an upper radial stand off on the inner surface of the housing (10);
c. a reservoir within the bypass housing (52) comprising a single dose of a medicament;
d. a guard (42) having an internal distal face and a drive tooth on an inner surface, where the drive tooth is slidably engaged with a track on the outer surface of the bypass housing (52);
e. a lower hub slidably engaged with the outer surface of the bypass housing (52) and slidably engaged with the inner surface of the guard (42); and
f. at least one biasing member (48).

2. The medicated module (4, 100) of claim 1 where at least one biasing member (48, 103) is engaged with a magnet member on a secondary housing (90, 130) when the medicated module (4, 100) is in proximity to the secondary housing (90, 130).

3. The medicated module (4, 100) of claim 2 where the biasing member (48, 103) is a spring.

4. The medicated module (4, 100) of claim 3 wherein when the biasing member (48, 103) is engaged with the secondary housing magnetic element (92), the biasing member magnetic element (82) extends toward the secondary housing magnetic element (92), thereby blocking the guard (84) from axial movement.

5. The medicated module (4, 100) of claim 1 wherein in a first position, the biasing member (48, 133) extends from the interior surface of the module (4, 100) into a rotary element (104), obstructing the rotation of the rotary element (104).

6. The medicated module (4, 100) of claim 5 further comprising a drug delivery device (110) with a magnetic element (114), wherein the drug delivery device (110) may be insertable into the medicated module (4, 100).

7. The medicated module (4, 100) of claim 6 wherein when the drug delivery device (110) is inserted into the medicated module (4, 100), the drug delivery device magnetic element (114) pulls the biasing member (48, 133) toward the drug delivery device (110) and away from the rotary element (104).

8. The medicated module (4, 100) of claim 7 wherein in a second position the biasing member (48, 133) is completely removed from the rotary element (104), allowing the rotary element (104) to rotate.

9. The medicated module (4, 100) of claim 8 wherein when the rotary element (104) is allowed to rotate, the medicated module (4, 100) is in a triggerable state.

10. The medicated module (4, 100) of claim 5 wherein when the medicated module (4, 100) is in the first position, the medicated module (4, 100) is in a trigger locked state.

11. The medicated module (4, 100) of claim 1 where the medicament comprises a fluid medicament.

12. The medicated module (4, 100) of claim 1 where the medicament comprises at least one of a GLP-1, a Insulin, or a mixture thereof.

13. A drug delivery system, comprising,
a. a medicated module (4, 100) with a module hole comprising,
i. a reservoir comprising a dose of a medicament with a bypass housing (52); and
ii. a needle guard (42) having an internal proximal face, the medicated module attachable to a drug delivery device (110);
b. a secondary packaging (90, 130) comprising,
i. at least one biasing member (81, 133) with a first end and a second end, wherein the first end is engaged with the internal surface of the secondary packaging (90, 130), and the second end is operably coupled to a magnetic element (82, 132), wherein the secondary packaging is configured to at least partially receive the medicated module

14. The drug delivery system of claim 13, wherein in a first position the magnetic element (82, 132) engages the medicated module (4, 100).

15. The drug delivery system of claim 14, wherein in a second position, a drug delivery device (110) with a magnetic element is engaged with the medicated module (4, 100), and repels the secondary packaging magnetic element (92), releasing the secondary packaging magnet element (82, 132) from its connection to the medicated module (4, 100).
